# EUROPÄISCHE PATENTSCHRIFT

(11) **EP 1 182 978 B1**
(45) Veröffentlichungstag und Bekanntmachung des Hinweises auf die Patenterteilung: **13.10.2004**
(21) Anmeldenummer: 00905064.2
(22) Anmeldetag: 14.02.2000
(51) Int. Cl.: A61B 17/72

(54) **AKTIVER MARKNAGEL ZUR DISTRAKTION VON KNOCHENTEILEN**
ACTIVE INTRAMEDULLARY NAIL FOR THE DISTRACTION OF BONE PARTS
CLOU D'OSTEOSYNTHESE INTRAMEDULLAIRE ACTIF DESTINE A L'ECARTEMENT DE PARTIES D'OS

(30) Priorität: 16.02.1999 DE 19906423
(43) Veröffentlichungstag der Anmeldung: 06.03.2002
(73) Patentinhaber: Wittenstein AG, 97999 Igersheim (DE)
(72) Erfinder: STAUCH, Roman, D-97959 Assamstadt (DE); KLEIN, Jürgen, D-97990 Weikersheim (DE)
(74) Vertreter: Weiss, Peter, Dr.
(86) Internationale Anmeldenummer: PCT/EP2000/001208
(87) Internationale Veröffentlichungsnummer: WO 2000/048524

(56) Entgegenhaltungen:
- EP-A- 0 306 443
- WO-A-96/25117
- US-A- 3 976 060
- US-A- 5 626 579

## Beschreibung

Die vorliegende Erfindung betrifft einen aktiven Marknagel mit ggf. anschliessender Prothese zur Distraktion von Knochensegmenten mit zwei gegeneinander bewegbaren Elementen und mit zumindest einem elektrisch betriebenen Antriebselement.

Derartige Marknägel sind in vielfältigster Form und Ausführungen auf dem Markt bekannt und gebräuchlich. Immer häufiger werden elektrische Antriebselemente in den Marknägeln vorgesehen, um eine Distraktion zweier gegeneinander bewegbarer Elemente zu erzeugen. Hierzu sind elektrische Anschlüsse notwendig um den Marknagel in Betrieb zu setzen.

Nachteilig an den bisher bekannten Anschlüssen ist, dass diese direkt in den Marknagel eingebettet sind und häufig durch mehrfaches Bewegen brechen oder beschädigt werden, wenn über einen bestimmten Zeitraum eine Distraktion erfolgt, oder auch durch äussere Einwirkungen, z.B. Bewegung der Gliedmassen.

Ferner ist nachteilig, dass diese Anschlüsse nach einer bestimmten Distraktion bspw. operativ oder durch Durchtrennen entfernt werden. Dann muss bei einer erneuten Aufnahme einer Distraktion dieser Anschluss operativ aufwendig angeschlossen werden.

Nach der WO 96/25 117 ist eine Distraktionsvorrichtung bekannt, bei welcher ein elektrisch betriebener Motor eine Spindel zur Bewegung einer Distraktionsvorrichtung in Betrieb setzt. Der elektrisch betriebene Motor wird über eine permanent angeschlossene Energiequelle in Betrieb genommen.

Die EP 0 306 443 A2 beschreibt einen Koaxialstecker für Herzschrittmacher, an welchem ein Anodenleiter sowie ein Katodenleiter anschliessen. Dieser Koaxialstecker ist mit einem Herzschrittmacher verbindbar.

Der vorliegenden Erfindung liegt die Aufgabe zugrunde, einen aktiven Marknagel zur Distraktion sowie einen Marknagel mit ggfs. anschliessenden Prothesen od. dgl. zu schaffen, mit welchem auf sehr einfache Weise eine elektrische Verbindung hergestellt werden kann. Diese soll den elektrischen, mechanischen und hygienischen Anforderungen gerecht werden.

Zur Lösung dieser Aufgabe führt, dass dem Marknagel die elektrische Energie über zumindest ein wiederlösbares Steckerelement zuführbar ist.

Bevorzugt endseits ist in einem der beiden axial gegeneinander bewegbaren Elemente des Marknagels eine Buchse in einer Öffnung vorgesehen. Stirnseitig kann dort in eine Öffnung ein Steckerelement eingesteckt werden, um eine elektrische Verbindung zur Steuerung und zur Energieübertragung herzustellen. Dabei können durchaus normierte Stecker bzw. Steckverbindungen verwendet werden, wie sie bspw. in der DIN-ISO 5841-3 beschrieben sind. Die bipolare Ausführung ist bevorzugt. Es können mehrere elektrische Signale übertragen werden.

Als besonders vorteilhaft hat sich erwiesen, dass dem Steckerelement vorzugsweise endseits zumindest ein Dichtlippenelement zugeordnet ist. Dieses Dichtlippenelement ist elastisch und bevorzugt nach aussen verjüngt ausgebildet. Es verschliesst die Öffnung der Buchse vollständig und absolut dicht. Hierdurch kann keine Gewebeflüssigkeit od. dgl. eintreten, um die elektrische Verbindung zwischen Buchse und Steckerelement zu beeinträchtigen.

Ferner ist von Vorteil, bei der vorliegenden Erfindung sollte der Marknagel als Prothese ausgebildet sein, dass nach einer gewünschten Distraktion lediglich die elektrische Steckverbindung operativ herausgelöst wird. Die Öffnung kann dann mit einem Propfen od. dgl. verschlossen werden. Sollte eine weitere Distraktion erforderlich sein, so kann operativ der Propfen wieder entfernt und dann der Stecker dort eingesteckt werden. Dies schafft erhebliche Vorteile insbesondere beim Operieren und Handhaben derartiger aktiver Marknägel.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Ansicht auf einen zumindest teilweise aufgeschnittenen aktiven Marknagel;
Figur 2a einen zumindest teilweisen Längsschnitt durch den Marknagel gemäss Figur 1;
Figur 2b einen Querschnitt durch den Marknagel entlang Linie II-II gemäss Figur 2a;
Figur 3a einen Teillängsschnitt durch den Marknagel gemäss Figur 1 als weiteres Ausführungsbeispiel;
Figur 3b einen Querschnitt durch den Marknagel gemäss Figur 3a entlang Linie III-III;
Figur 4 einen zumindest teilweise dargestellten Teillängsschnitt durch ein weiteres Ausführungsbeispiel eines Marknagels gemäss Figur 1.

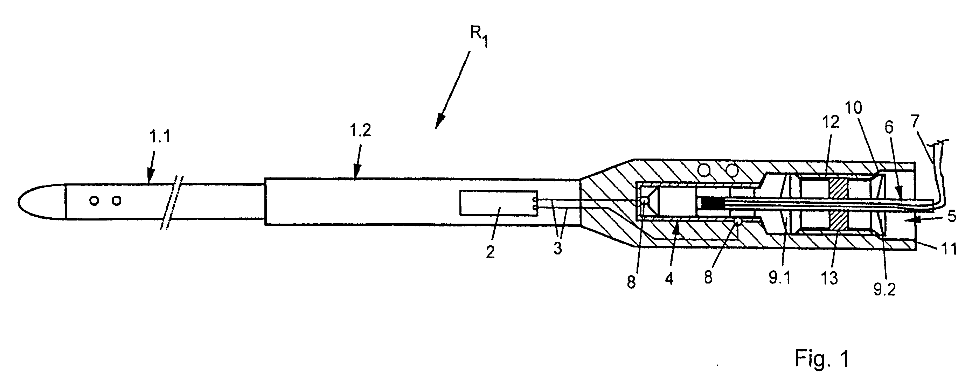

Gemäss Figur 1 weist ein erfindungsgemässer aktiver Marknagel R₁ zwei axial gegeneinander bewegbare Elemente 1.1, 1.2 auf. Im bevorzugten Ausführungsbeispiel ist das Element 1.1 gegenüber dem Element 1.2 verfahrbar. Hier nicht dargestellte Antriebsmittel, wie bspw. Getriebe, Schubstangen, Formgedächtnisantriebe od. dgl. können das Element 1.1 gegenüber dem Element 1.2 bewegen. Vorzugsweise ist im Element 1.2 des Marknagels R₁ zumindest ein elektrisches Antriebselement 2 vorgesehen. Dieses steht über elektrische Verbindungsleitungen 3 mit einer Buchse 4 des Elementes 1.2 in Verbindung. Die Buchse 4 ist stirnund endseitig des Elementes 1.2 im Marknagel vorgesehen. Durch eine Öffnung 5 lässt sich ein Steckerelement 6 in die Buchse 4 einschieben. An das Steckerelement 6 schliessen Energieversorgungsleitungen 7 an, welche bspw. zu einer externen Energiequelle führen. Von dort kann Energie direkt oder indirekt bspw. induktiv eingeleitet werden. Diese Energie ist zum Betreiben und Steuern des Antriebselementes 2 erforderlich.

Damit über einen gewissen Zeitraum, während der aktive Marknagel R₁ im Knochen verbleibt, eine gewünschte Distraktion erfolgen kann, ist es erforderlich eine elektrische Verbindung permanent herzustellen. Dabei hat sich als besonders vorteilhaft erwiesen stirnseitig, insbesondere endseitig in das Element 1.2 ein sogenanntes Steckerelement 6 einzustecken. Entsprechende Kontaktstellen 8 übertragen elektrische Energie und/oder elektrische Signale.

Wichtig bei derartigen Verbindungen ist jedoch, dass aus hygienischen Gründen eine absolute dichte saubere wiederlösbare Verbindungsstelle zwischen Marknagel R₁ und Steckerelement 6 hergestellt wird. Hierzu wird zumindest ein Dichtlippenelement 9.1, 9.2 dem Steckerelement 6 zugeordnet, welches die Öffnung 5 und damit die Buchse 4 des Elementes 1.2 wiederlösbar verschliesst. Hierdurch bleibt gewährleistet, dass über lange Zeiträume immer ein elektrischer Kontakt wiederlösbar hergestellt bleibt.

Ausserdem gewährleistet das äussere Dichtlippenelement 9.2 einen optimalen Knickschutz, welcher bei den vorhandenen Biegewechselbelastungen von Bedeutung ist.

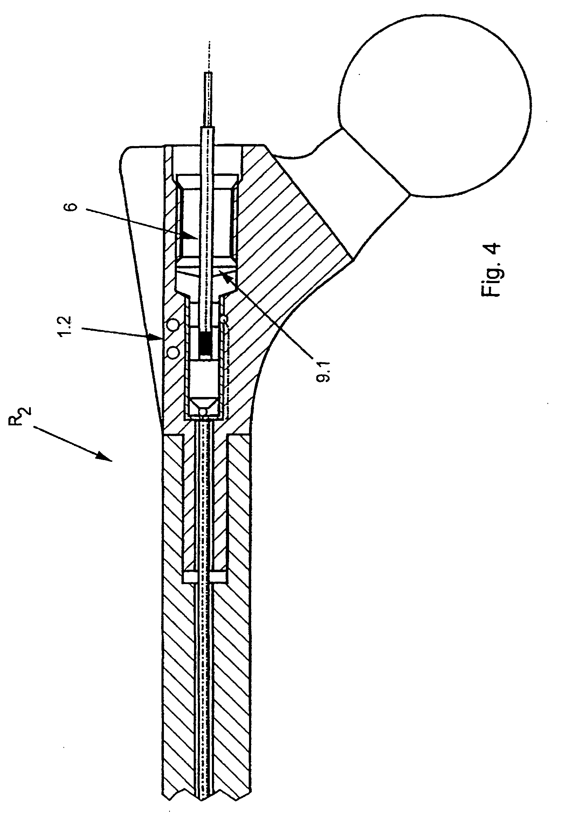

Ist bspw. der aktive Marknagel eine Hüftgelenksendprothese, wie sie in Figur 4 teilweise dargestellt ist, oder eine Kniegelenksendprothese, so kann diese ebenfalls, wie oben beschrieben, mit einer entsprechenden Steckverbindung vorgesehen sein. Hierdurch lässt sich bspw. nach der Operation durch eine entsprechende Distraktion eine Korrektur vornehmen. Dies kann über einen längeren Zeitraum der Genesung geschehen. Ist dann bspw. keine Korrektur mehr erforderlich so wird lediglich das Steckerelement aus der Öffnung 5 des Elementes 1.2 entfernt. Gegebenenfalls wird die Öffnung 5 mit einem dichten Propfenverschluss od. dgl. verschlossen, sollte nochmals ein Korrektur erforderlich sein. Auf diese Weise lassen sich als aktive Marknägel R₁ ausgebildete Prothesen permanent bedienen, wobei nach einer entsprechenden Distraktion oder Korrektur des Knochens ein Entfernen der elektrischen Anschlüsse ohne weiteres möglich ist.

Sollte nach einer bestimmten Dauer eine weitere Korrektur erforderlich werden, so kann das Steckerelement wieder in die Öffnung eingeführt werden. Eine ggfs. vollimplantierbare subkutane Energiequelle, an welche Engergie oder entsprechende Signale induktiv weitergegeben werden, kann dann unter der Haut implantiert werden. Der aktive Marknagel kann auf diese Weise wieder in Betrieb genommen werden.

Von Bedeutung ist auch ein radiales Dichtlippenelement 9.1, 9.2, welches ggfs. an einem nach innen ragenden Absatz 10 anliegt. Bevorzugt ist das Dichtlippenelement 9.1, 9.2 nach aussen verjüngt ausgebildet und liegt dicht an einer Innenwand 11 der Buchse 4 bzw. des Elementes 1.2 an.

Es ist ferner daran gedacht ein Innengewinde 12 innerhalb der Buchse 4 vorzusehen, in welche ein entsprechendes Aussengewinde 13 des Steckerelementes 6 eingreift, um das Steckerelement 6 wiederlösbar festzulegen. Bevorzugt werden jedoch Rastverbindungen od. dgl. verwendet.

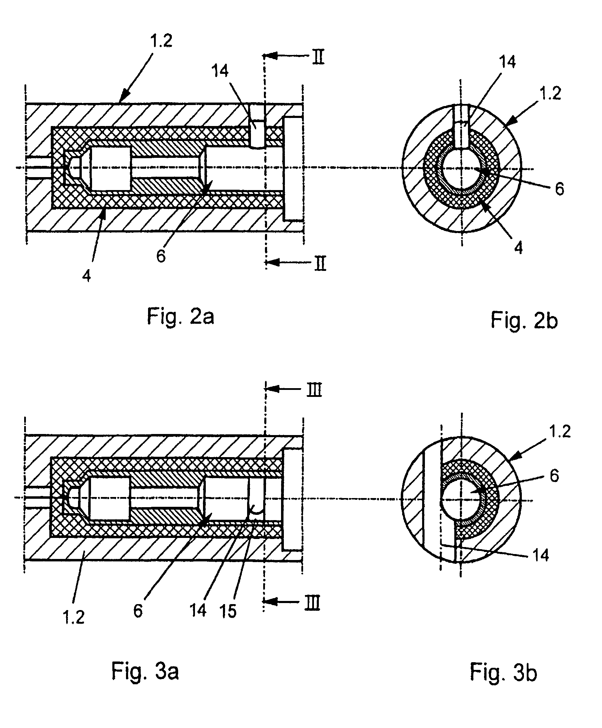

Als Sicherung gegen ein Herausziehen im Betrieb können einfache Gewindestifte od. dgl. vorgesehen sein, wie sie bspw. in Figur 2a aufgezeigt sind. Dort durchgreift ein entsprechendes Sicherungselement 14 als Gewindestift radial das Element 1.2, um das in der Buchse 4 eingesteckte Steckerelement 6 wiederlösbar festzulegen bzw. festzuklemmen. Dies wird auch in der querschnittlichen Darstellung gemäss Figur 2b verdeutlicht.

Ein weiteres Sicherungselement 14 zeigt die Figuren 3a und 3b, welches in eine entsprechende Nut 15 des Steckerelementes 6 eingreift und hierdurch eine axiale Sicherung des Steckerelementes 6 gegenüber der Buchse 4 des Elementes 1.2 bildet. Dabei kann das Sicherungselement 14 als Riegel od. dgl. vorgesehen sein, um ein axiales Bewegen des Steckerelementes 6 gegenüber der Buchse 4 zu verhindern. Hier sei der Erfindung keine Grenze gesetzt.

In dem letzten Ausführungsbeispiel gemäss Figur 4 ist verdeutlicht, dass auch in beliebige Prothesen, Hüftgelenksendprothesen als aktive Marknägel R₂ entsprechende Steckerelemente 6, in oben beschriebener Weise, eingesetzt sein können. Dies soll auch im Rahmen der vorliegenden Erfindung liegen.

| **Positionszahlenliste** | | | | | |
|---|---|---|---|---|---|
| 1 | Element | 34 | | 67 | |
| 2 | Antriebselement | 35 | | 68 | |
| 3 | Verbindungsleitung | 36 | | 69 | |
| 4 | Buchse | 37 | | 70 | |
| 5 | Öffnung | 38 | | 71 | |
| 6 | Steckerelement | 39 | | 72 | |
| 7 | Verbindungsleitung | 40 | | 73 | |
| 8 | Kontaktstelle | 41 | | 74 | |
| 9 | Dichtlippenelement | 42 | | 75 | |
| 10 | Absatz | 43 | | 76 | |
| 11 | Innenwand | 44 | | 77 | |
| 12 | Innengewinde | 45 | | 78 | |
| 13 | Aussengewinde | 46 | | 79 | |
| 14 | Sicherungselement | 47 | | | |
| 15 | Nut | 48 | | R₁ | aktiver Marknagel |
| 16 | | 49 | | R₂ | aktiver Marknagel |
| 17 | | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Aktiver Marknagel mit ggf. anschliessender Prothese zur Distraktion von Knochensegmenten mit zwei gegeneinander bewegbaren Elementen (1.1, 1.2) und mit zumindest einem elektrisch betriebenen Antriebselement (2),
**dadurch gekennzeichnet,**
**dass** dem Marknagel die elektrische Energie über zumindest ein wiederlösbares Steckerelement (6) zuführbar ist.

2. Aktiver Marknagel nach Anspruch 1, **dadurch gekennzeichnet, dass** das wiederlösbare Steckerelement (6) stirnseitig in eine Öffnung (5) des Marknagels einsetzbar ist.

3. Aktiver Marknagel nach Anspruch 1 oder 2, **dadurch gekennzeichnet, dass** das Steckerelement (6) in eine Buchse (4) des Marknagels wiederlösbar eingreift und hierdurch zumindest eine elektrische Kontaktstelle (8) bildet.

4. Aktiver Marknagel nach wenigstens einem der Ansprüche 1 bis 3, **dadurch gekennzeichnet, dass** dem Steckerelement (6) zumindest ein Dichtlippenelement (9.1, 9.2) zugeordnet ist.

5. Aktiver Marknagel nach Anspruch 4, **dadurch gekennzeichnet, dass** das zumindest eine Dichtlippenelment (9.1, 9.2) radial am Steckerelement (6) angeordnet ist.

6. Aktiver Marknagel nach Anspruch 4 oder 5, **dadurch gekennzeichnet, dass** das zumindest eine Dichtlippenelement (9.1, 9.2) nach aussen verjüngt ausgebildet ist.

7. Aktiver Marknagel nach wenigstens einem der Ansprüche 4 bis 6, **dadurch gekennzeichnet, dass** das Dichtlippenelement (9.1, 9.2) die Öffnung (5) des Elementes (1.2) des Marknagels stirnseitig verschliesst, wobei das Dichtlippenelement (9.1, 9.2) ggfs. an einem inneren Absatz (10) anliegt.

8. Aktiver Marknagel nach wenigstens einem der Ansprüche 1 bis 7, **dadurch gekennzeichnet, dass** das Steckerelement (6) über ein Sicherungselement (14) wie Gewindestifte, Riegelelemente od. dgl. in der Öffnung (5) des Marknagels vor Herauslösen gesichert ist.

9. Aktiver Marknagel nach Anspruch 8, **dadurch gekennzeichnet, dass** das Sicherungselement (14) axial in das Steckerelement (6) zumindest teilweise eingreift und dieses gegenüber dem Element (1.2) des Marknagels wiederlösbar verspannt.

10. Aktiver Marknagel nach wenigstens einem der Ansprüche 2 bis 9, **dadurch gekennzeichnet, dass** innerhalb der Öffnung (5) ein Innengewinde (12) vorgesehen ist, in welches zur Festlegung und Fixierung des Steckerelementes (6) ein entsprechend passendes Aussengewinde (13) eingreift.

11. Aktiver Marknagel nach wenigstens einem der Ansprüche 1 bis 10, **dadurch gekennzeichnet, dass** an das Steckerelement (6) eine Energiequelle, insbesondere eine induktive vollimplantierbare Energiequelle zur induktiven Energieübertragung ggfs. subkutan anschliessbar ist.

## Claims

1. Active intramedullary nail, possibly with a prosthesis communicating therewith, for distracting bone segments, said nail having two elements (1.1, 1.2) which are displaceable relative to each other, and having at least one electrically driven drive member (2), **characterised in that** the electrical power can be supplied to the intramedullary nail via at least one re-releasable plug-in element (6).

2. Active intramedullary nail according to claim 1, **characterised in that** the re-releasable plug-in element (6) is insertable, at the end face, into an opening (5) of the intramedullary nail.

3. Active intramedullary nail according to claim 1 or 2, **characterised in that** the plug-in element (6) engages in a bush (4) of the intramedullary nail in a re-releasable manner and hereby forms at least one electrical contact point (8).

4. Active intramedullary nail according to at least one of claims 1 to 3, **characterised in that** at least one sealing lip element (9.1, 9.2) is associated with the plug-in element (6).

5. Active intramedullary nail according to claim 4, **characterised in that** the at least one sealing lip element (9.1, 9.2) is disposed radially on the plug-in element (6).

6. Active intramedullary nail according to claim 4 or 5, **characterised in that** the at least one sealing lip element (9.1, 9.2) has an outwardly tapering configuration.

7. Active intramedullary nail according to at least one of claims 4 to 6, **characterised in that** the sealing lip element (9.1, 9.2) closes, at the end face, the opening (5) of the element (1.2) of the intramedullary nail, the sealing lip element (9.1, 9.2) possibly abutting against an inner shoulder portion (10).

8. Active intramedullary nail according to at least one of claims 1 to 7, **characterised in that** the plug-in element (6) is prevented from being released via a securement member (14), such as threaded pins, locking members or the like, in the opening (5) of the intramedullary nail.

9. Active intramedullary nail according to claim 8, **characterised in that** the securement member (14) engages axially in the plug-in element (6), at least partially, and clamps said plug-in element in a re-releasable manner relative to the element (1.2) of the intramedullary nail.

10. Active intramedullary nail according to at least one of claims 2 to 9, **characterised in that** an internal thread (12) is provided internally of the opening (5), in which thread an appropriately fitting external thread (13) engages to fasten and secure the plug-in element (6).

11. Active intramedullary nail according to at least one of claims 1 to 10, **characterised in that** a power source, more especially an inductive, fully implantable power source for transmitting inductive power, is possibly subcutaneously connectable to the plug-in element (6).

## Revendications

1. Clou d'ostéosynthèse intramédullaire actif, avec éventuellement une prothèse qui le suit, destiné à l'écartement de segments d'os avec deux éléments (1.1, 1.2) mobiles l'un par rapport à l'autre, avec au moins un élément d'entraînement à commande électrique (2), **caractérisé par le fait que** l'énergie électrique peut être alimentée vers le clou d'ostéosynthèse intramédullaire par l'intermédiaire d'au moins d'un élément enfichable (6) amovible.

2. Clou d'ostéosynthèse intramédullaire actif selon la revendication 1, **caractérisé par le fait que** l'élément enfichable (6) amovible peut être placé, du côté frontal, dans une ouverture (5) du clou d'ostéosynthèse intramédullaire.

3. Clou d'ostéosynthèse intramédullaire actif selon la revendication 1 ou 2, **caractérisé par le fait que** l'élément enfichable (6) s'engage, de manière amovible, dans une douille (4) du clou d'ostéosynthèse intramédullaire et forme ainsi au moins un point de contact électrique (8).

4. Clou d'ostéosynthèse intramédullaire actif selon au moins l'une des revendications 1 à 3, **caractérisé par le fait qu'**à l'élément enfichable (6) est associé au moins un élément de lèvre d'étanchéité (9.1, 9.2).

5. Clou d'ostéosynthèse intramédullaire actif selon la revendication 4, **caractérisé par le fait que** l'au moins un élément de lèvre d'étanchéité (9.1, 9.2) est disposé radialement sur l'élément enfichable (6).

6. Clou d'ostéosynthèse intramédullaire actif selon la revendication 4 ou 5, **caractérisé par le fait que** l'au moins un élément de lèvre d'étanchéité (9.1, 9.2) est réalisé effilé vers l'extérieur

7. Clou d'ostéosynthèse intramédullaire actif selon au moins l'une des revendications 4 à 6, **caractérisé par le fait que** l'élément de lèvre d'étanchéité (9.1, 9.2) obture, du côté frontal, l'ouverture (5) de l'élément (1.2) du clou d'ostéosynthèse intramédullaire, l'élément de lèvre d'étanchéité (9.1, 9.2) s'appuyant éventuellement sur un décrochement intérieur (10).

8. Clou d'ostéosynthèse intramédullaire actif selon au moins l'une des revendications 1 à 7, **caractérisé par le fait que** l'élément enfichable (6) est, par l'intermédiaire d'un élément de sécurité (14) tel que des goupilles filetées, des éléments de verrou ou autres, empêché de sortir de l'ouverture (5) du clou d'ostéosynthèse intramédullaire.

9. Clou d'ostéosynthèse intramédullaire actif selon la revendication 8, **caractérisé par le fait que** l'élément de sécurité (14) s'engage axialement, du moins partiellement, dans l'élément enfichable (6) et serre ce dernier, de manière amovible, par rapport à l'élément (1.2) du clou d'ostéosynthèse intramédullaire.

10. Clou d'ostéosynthèse intramédullaire actif selon au moins l'une des revendications 2 à 9, **caractérisé par le fait qu'**à l'intérieur de l'ouverture (5) est prévu un filet intérieur (12) dans lequel s'engage, pour la fixation de l'élément enfichable (6), un filet extérieur (13) correspondant.

11. Clou d'ostéosynthèse intramédullaire actif selon au moins l'une des revendications 1 à 10, **caractérisé par le fait qu'**à l'élément enfichable (6) peut être raccordée de manière sous-cutanée, une source d'énergie, en particulier une source d'énergie inductive entièrement implantable destinée à la transmission d'énergie inductive.
